Europäisches Patentamt

European Patent Office

Office européen des brevets

(11) Publication number: **0 270 846 B1**

# EUROPEAN PATENT SPECIFICATION

(45) Date of publication of patent specification: **11.12.91**   (51) Int. Cl.⁵: **A61L 15/00, D04H 1/54, //A61F13/15**

(21) Application number: **87116382.0**

(22) Date of filing: **06.11.87**

(54) **Absorbent article.**

(30) Priority: **10.12.86 JP 294425/86**

(43) Date of publication of application:
**15.06.88 Bulletin 88/24**

(45) Publication of the grant of the patent:
**11.12.91 Bulletin 91/50**

(84) Designated Contracting States:
**DE ES FR GB**

(56) References cited:
**EP-A- 0 070 164        EP-A- 0 109 280**
**EP-A- 0 171 807        EP-A- 0 209 775**
**GB-A- 2 100 130        US-A- 4 480 000**

(73) Proprietor: **Kao Corporation**
**14-10, Nihonbashi Kayabacho 1-chome**
**Chuo-Ku Tokyo 103(JP)**

(72) Inventor: **Shiba, Daisuke**
**4254-14, Hiraidecho**
**Utsunomiya-shi Tochigi(JP)**
Inventor: **Amanoi, Akira**
**B-202, 396-12, Minemachi**
**Utsunomiya-shi Tochigi(JP)**

(74) Representative: **Dickel, Klaus, Dipl.-Ing. et al**
**Julius-Kreis-Strasse 33**
**W-8000 München 60(DE)**

## Description

(Field of Industrial Application)

The present invention relates to a disposable absorbent article using a non-woven fabric having excellent absorptivity and processability as the surface material, and particularly to an absorbent article such as a sanitary napkin, a paper diaper or a sheet for makeup.

(Prior Art and Problems to be Solved)

A conventional absorbent article such as a sanitary napkin or a paper diaper, as known from US-A-4,519,799 comprises an absorbent layer made of cottony pulp or absorbent paper, a leakproof layer disposed on the lower and lateral sides of the absorbent layer, and a non-woven fabric provided on the surface of the absorbent layer.

The absorptivity of the absorbent article has been greatly improved in recent years since new materials such as a super absorbent polymer and a dry process non-woven fabric have been introduced with rapid progress of the related techniques. Particularly as to the non-woven fabric, a synthetic fiber has begun to be predominantly used as the material of the non-woven fabric in place of a regenerated fiber of cellulose which has heretofore been widely used, because the synthetic fiber is very effective in suppressing the tackiness of the surface of the non-woven fabric.

An absorbent article comprising a combination of materials each having excellent absorptivity cannot be said to sufficiently exhibit the innate performance in actual use. This is apparent from the fact that the most serious dissatisfaction of the consumers with an absorbent article such as a sanitary napkin or a paper diaper is still "leakage" from the crotch of a wearer.

The principal causes of the leakage are separation of each constituent materials and large slippage or wrinkling of the absorbent article which are due to irregular stress applied to the absorbent article when the crotch of the wearer is moved. Among others, particularly the non-woven fabric receives a large stress since it is directly contacted with the skin of the wearer so that it is liable to be separated from a waterproof paper or the absorbent layer. Thus, integration of them by any method is earnestly desired.

As a means of integration of the non-woven fabric with the waterproof paper or the absorbent layer, it is conceivable to bond them with a pressure-sensitive adhesive or a hot-melt adhesive. If such a means is employed, however, the production steps are very complicated, inevitably largely increasing the production cost. In contrast, if a so-called heat bonding method, in which a non-woven fabric is molten and bonded to an object by simple hot-pressing, can be employed, complicatedness of the steps is minimized, and high-speed production becomes possible with a slight increase in the production cost.

In view of the above, a non-woven fabric having a good thermal processability is necessary for improving the leakproofness of an absorbent article under conditions of movement of the wearer while not increasing the cost so much. For high-speed production of such an absorbent article, other excellent processabilities are required of the non-woven fabric in addition to the thermal processability.

However, a synthetic resin non-woven fabric which has been predominantly used has very insufficient processabilities though it has excellent absorptivity. The problems can be broadly summarized in the following two ones.

A first problem is that thermal processing of the non-woven fabric is difficult. When the non-woven fabric is made of a fiber incapable of being heat-molten, such as an acrylic fiber, the fabric cannot be bonded to an object at all because the non-woven fabric cannot be molten. When a non-woven fabric made of a polyester fiber, a nylon fiber, or the like can be molten by heat but the melting temperature is very high, control of the temperature is difficult because of a very high processing temperature, and a damage to other materials may be large when the molten non-woven fabric contacts with them. Thus, even when the non-woven fabric is molten in processing, the following difficulties are experienced.

The molten fiber sticks to a heat sealer, with the result that the heat bonded portion of the non-woven fabric is broken and that the function of the sealer is deteriorated because of the resin sticking to the sealer. Even when a low-melting fiber is partially blended in a non-woven fabric in order to lower the processing temperature, the situation is the same if all of the low-melting fibers are molten. In most cases, the low-melting fiber used herein is one comprising components all having the same melting temperature, such as a polypropylene fiber, or one comprising components having a small difference in melting temperature from one another, such as a polyethylene/polypropylene conjugate fiber. When processing is conducted at a high temperature enough to provide sufficient heat-bonding strength, all components are molten at the same time, and the molten component instantly migrates to the sealer, leading to a reduction in the function of the sealer and to the breakage of the heat-bonded portion. As the blending rate of the low-melting fiber is increased, this problem is more serious. When the blending rate of the low-melting fiber is low, migration of the molten component to the sealer can be somewhat prevented by bonding and intertwining of the low-melting fiber with the high-melting fiber in a temperature range wherein all of the low-melting fibers are molten but the high-melting fibers keep the fibrous form. However, the effect of thermal adhesion is hardly exhibited because of the low blending rate of the molten component, and migration of the molten component to the sealer remains yet to be eliminated. Thus, the breakage of the heat-bonded portion of the non-woven fabric and damages caused by the deposited matter accumulated in the sealer by running the sealer particularly for a long time cannot be essentially obviated.

There has been an attempt to improve the above-mentioned problems by using as the low-melting fiber a polyester/polyester conjugate fiber having a large difference in the melting temperature between the two component fibers. In this case, a conventional low-melting polyester component is so amorphous that it does not have a melting temperature (but has a softening point) in a strict sense. Thus, the adhesion to an object is low and hence no sufficient sealing strength can be obtained even at a temperature above the softening point.

As described above, thermal processing of a non-woven fabric based on a synthetic fiber can merely be conducted in a very restricted range. Particularly, it is impossible to conduct thermal processing which will exert a consistent effect on a non-woven fabric having a very high blending rate of a low-melting fiber used as the binder, such as a dry process heat-bonded non-woven fabric which is widely used in absorbent articles.

Thus, in the most widely employed method of providing a thermal processability to a synthetic fiber-based non-woven fabric, a polyolefin fiber or its conjugate fiber having a relatively high bonding strength when thermally processed is blended within a range wherein the fiber is not so much fused to a sealer. However, this method can not essentially solve the above-mentioned problem, and involves the following serious problem.

Specifically, a second problem is that, when a polyolefin fiber is used, the cuttability of a non-woven fabric is lowered. In production of a disposable absorbent article such as a sanitary napkin or a paper diaper, the step of cutting a product or a raw material does not fail to be included in the process. Generally speaking, the cuttability of a synthetic fiber is poor as compared with that of a regenerated cellulose fiber such as rayon. A high-speed productivity is required of the above-mentioned absorbent article for lowering the cost. Thus, cutting must be done in a very short time and the cutting system is limited. In a cutting system using a metallic blade, which is usually employed, the durability of the blade is greatly affected by the properties of materials such as a non-woven fabric. On the side of the non-woven fabric, it is not preferred that the fabric undergo damages to a portion other than a cut portion by too hard a cutting treatment. It is believed that the cuttability has something to do with the viscoelasticity of a fiber. The cuttability of a polyolefin fiber having a high viscoelasticity is particularly poor, while those of a polyester fiber or an acrylic fiber having higher brittleness are relatively good. Accordingly, a dry process heat-bonded non-woven fabric based on a polyolefin fiber and a non-woven fabric containing a polyolefin fiber blended for providing thermal processability are difficult to process. Thus, a difficulty is encountered in high-speed production of an absorbent article.

As described above, a conventional non-woven fabric based on a synthetic fiber does not simultaneously satisfy sufficient thermal processability and cuttability under conditions of high-speed production. Thus, no absorbent article which hardly slips or wrinkles and has a high leakproofness has been obtained.

[Means for Solving the Problems]

The inventors of the present invention have made intensive investigations with a view to providing a non-woven fabric having sufficient thermal processability and cuttability, high strength and absorptivity, and good hand. As a result, they have completed the absorbent article of the present invention.

Specifically, the present invention provides an absorbent article comprising, as the surface material, a non-woven fabric containing 40 wt.% or more of a conjugate fiber made of a first polyester and a second polyester having a melting temperature of 50°C or more below that of said first polyester and a height of an endothermic peak of 5 % or more of that of the first polyester.

When the first problem as summarized in the preceding item is considered, the non-woven fabric to be used in the absorbent article of the present invention must satisfy at least the following conditions in thermal processing.

A first condition is that at least part of the non-woven fabric is molten and efficiently bonded to an object under heat and pressure. This is obvious when the purpose of the invention is considered. A second condition is that the component of the non-woven fabric molten under heat and pressure does not migrate to a sealer. A third condition is that the above-mentioned conditions can be satisfied in a wide temperature range. Particularly, the second and third conditions are quite important requisites in conducting stable thermal processing in actual production.

As a result of investigation on these conditions, the inventors of the present invention have found that a non-woven fabric satisfying the above-mentioned conditions can be obtained by the following method.

First, in order to improve the efficiency of bonding of the non-woven fabric to an object, a component which not only is molten under heat and pressure, but also rapidly flows to reach the object after melting must be included in the non-woven fabric. The second polyester is the conjugate fiber according to the present invention corresponds to this component.

Second, the method of preventing migration of the component of the non-woven fabric to a sealer and breakage of the bonded portion will be described. This is attained by using a fiber wherein not all of resin components are molten under heat and pressure. Specifically, the second polyester is molten with adequate flowability at the time of heat bonding, while the first polyester is not molten and maintains the form of the fiber to serve as a skeleton of the non-woven fabric. A conjugate fiber as mentioned just above should be used for attaining the purpose. In order to certainly realize such an effect within a range of dispersion of the heat bonding temperature and the line in actual production, a difference in melting temperature between the first and second polyesters must be at least $50\,^{\circ}C$, desirably $100\,^{\circ}C$ or more. In such a conjugate fiber, as the melting temperature of the first polyester is higher, the thermal processing can be done in a wider temperature range.

In order that the non-woven fabric simultaneously satisfies the first and second conditions necessary as regards thermal processability, the above-mentioned conjugate fiber made of the first and second polyesters must be partially contained at least in the surface layer of the non-woven fabric. As the blending rate of the conjugate fiber in the surface layer is increased, the adhesion strength increases without breakage of the heat-bonded portion and fusion of the resin to the sealer, thus improving the thermal processability of the non-woven fabric.

In order that the non-woven fabric according to the present invention satisfies the third condition necessary for thermal processing, it is desired that the bonding by thermal processing occur at as low a temperature as possible while fusion to the sealer do not occur up to as high a temperature as possible. Namely, it is desired that the first polyester of the conjugate fiber according to the present invention have as high a melting temperature as possible, while the second polyester have as low a melting temperature as possible. The melting temperature of the first polyester is desirably $200\,^{\circ}C$ or higher, while that of the second polyester is desirably $180\,^{\circ}C$ or lower.

When attention is paid only to the thermal processability, examples of the conjugate fiber (first resin component/second resin component) satisfying the above-mentioned properties include polypropylene/low density polyethylene, polypropylene/ethylene-vinyl acetate copolymer, polyester/polyethylene, and polyester/amorphous polyester. Among them, conventional polyester/amorphous polyester provides such a poor bonding to an object that no sufficient sealing strength can be obtained, because the polyester as the second resin component has a low thermal flowability due to amorphousness.

The cuttability of the non-woven fabric as the second problem summarized in the preceding item is considered in addition to the thermal processability. The above-mentioned polyolefin-based conjugate fibers having good thermal processability have poor cuttability of a non-woven fabric as discussed in the preceding item. The conventional polyester/amorphous polyester conjugate fiber has a relatively good cuttability in contrast with the thermal processability.

The inventors of the present invention, based on the overall consideration of the above, have attempted to modify the polyester/amorphous polyester conjugate fiber to improve the thermal processability thereof. As a result, it has become possible to prepare a non-woven fabric which has a good thermal processability comparable to those of polyolefin-based conjugate fibers without detriment to the cuttability and which has excellent strength and hand which cannot be obtained by the conventional polyester/amorphous polyester conjugate fiber.

The aim of the modification is to impart an adequate thermal flowability, the bonding strength after heat melting and adequate melting temperature to the conventional amorphous polyester component. These physical properties have a complicated correlation with the molecular structure of the resin and the crystalline structure in a fibrous form. The inventors of the present invention have focused their attention mainly on the molecular weight of the resin component, the crystallinity in the fibrous form, and the like, and controlled them to realize the desired modification. They have found that the endothermic peak of the low-melting polyester component when molten by heat corresponds to flowability and bonding strength, and hence can serve as an index therefor. Specifically, it has been found that, when the molecular weight, crystallinity in the fibrous form, or the like of the second polyester are controlled to be increased in such a way as will increase the proportion of an endothermic peak of the second polyester when molten by heat relative to an endothermic peak of the first polyester, the flowability at the time of melting by heat and heat bonding strength are increased. In fact, the conventional amorphous polyester, which is poor in flowability when molten by heat and heat-bonding strength, has no melting temperature and a proportion of an endothermic peak thereof as mentioned above is 0%. In order to satisfy the above-mentioned three conditions necessary for thermal processing of the non-woven fabric, the height of the endothermic peak of the second polyester must be 5% or more, desirably 20% or more, of that of the first polyester.

Any polyester resin having a high melting temperature and a good cuttability may be used as the first polyester. Examples of the first polyester resin include not only the most common polyethylene terephthalate but also polybutylene terephthalate. The melting temperature of the second polyester must be designed to be by at least 50°C, desirably at least 100°C, lower than that of the first polyester.

A non-woven fabric having excellent heat processability and cuttability is formed from a conjugate fiber (hereinafter referred to as "PET/ImpPET") by the following procedure. Specifically, when the cuttablity of a fiber other than PET/ImpPET is good as in the case of a polyester fiber and an acrylic fiber, only good thermal processability must be imparted to the non-woven fabric constituted of that fiber. When the cuttability of a fiber other than PET/impPET is not good as in the case of a polyolefin fiber, the cuttability of such a fiber may be improved by blending PET/ImpPET in a non-woven fabric constituted of such a fiber.

In any case, inclusion of PET/ImpPET at least in every layer of the non-woven fabric is necessary. In order to secure a consistent effect, PET/ImpPET must be contained in an amount of 40 wt.% or more on the average in the non-woven fabric.

Besides the above-mentioned results, the PET/ImpPET according to the present invention enables production of a non-woven fabric having excellent strength and hand which cannot be obtained from the conventional polyester/amorphous polyester conjugate fiber.

Conditions for most effectively using the conjugate fiber according to the present invention in a dry process heat bonded non-woven fabric will now be described.

When consideration is given to the balance of the strength and hand of a non-woven fabric itself, at least the surface layer of the non-woven fabric comprises a conjugate fiber according to the present invention and a fiber having a melting temperature comparable to or higher than that of a second polyester of the conjugate fiber in an aspect of a composition. The average weight proportion of them is desirably in the range of 40 to 100 : 60 to 0. The areal weight is desired to be 10 to 40 g/m$^2$ for overall and 5 to 15 g/m$^2$ for the surface layer in the case of use in a sanitary napkin, and 20 to 50 g/m$^2$ foroverall and 7 to 20 g/m$^2$ in the case of use in a paper diaper. Particularly in order to obtain strength and hand comparable to those of a non-woven fabric based on a polyolefin fiber, the melt viscosity of the second polyester as the direct index of flowability is 5 poises or lower at 200°C. The fineness of the conjugate fiber according to the present invention may be 1.5 to 10 deniers. It is preferably 1.5 to 6 deniers when consideration is given to the strength and hand.

Lastly, in order that an absorbent article has an adequate absorptivity, an adequate hydrophilic nature is preferably imparted to a non-woven fabric. Thus, the conjugate fiber according to the present invention is desired to have a hydrophilic nature at least on its surface. Methods of making the surface hydrophilic include one in which the surface of the conjugate fiber is treated with a surfactant to make it hydrophilic, one in which a chemical substance having a hydrophilic group, such as a monomer having a hydrophilic group or a polymer having a hydrophilic group, is chemically bonded to the conjugate fiber to effect chemical surface modification, one using plasma processing, and one in which a chemical substance having a hydrophilic group is kneaded into the conjugate fiber to effect physical surface modification. In chemical surface modification, a chemical substance having a hydrophilic group may be chemically bonded to the fiber surface, or a chemical substance having a hydrophilic group may be bonded and crosslinked between the molecules thereof to cover the fiber surface. As described above, the methods of making the fiber hydrophilic during the fiber production step are generally employed. Alternatively, a non-woven fabric is formed and then post-treated to effect the above-mentioned chemical or physical surface modification, or the treatment with a surfactant, whereby the hydrophilic nature may be imparted to the surface of the conjugate fiber according to the present invention.

[Examples]

Absorbent articles thermally processed using a non-woven fabrio according to the present invention will now be described in more detail with reference to Examples.

Examples 1 to 12 and Comparative Examples 1 to 8 <fiber, non-woven fabric, and absorbent article>

Fibers used as conjugate fibers according to the present invention and fibers used as ones outside the scope of the present invention are summarized in Table 1.

Physical properties of non-woven fabrics prepared using these fibers and absorbent articles of the present invention and comparative absorbent articles using these non-woven fabrics are summarized in Table 2.

In Examples 1 to 5, Examples 9 to 12, Comparative Examples 1 to 3, and Comparative Examples 6 to 8, a non-woven fabric was prepared by the heat bonding method using a conjugated fiber as the binder fiber (a hot air of 140°C was passed through a card web to fuse the conjugated fiber to other fiber, thereby effecting fixation). In Examples 6 to 8 and Comparative Examples 4 and 5, a non-woven fabric was formed by ejecting a high-pressure water flow (propellent pressure: 55 kg/cm²) against a card web to intertwine fibers.

An absorbent article was produced by placing a non-woven fabric as mentioned in Table 2 instead of a non-woven fabric removed from a commercially available sanitary napkin (trade name: Lorie Safety Long (manufactured by Kao Soap Co., Ltd.)). Sealing and cutting operations were conducted as follows.

Brief Description of the drawings:

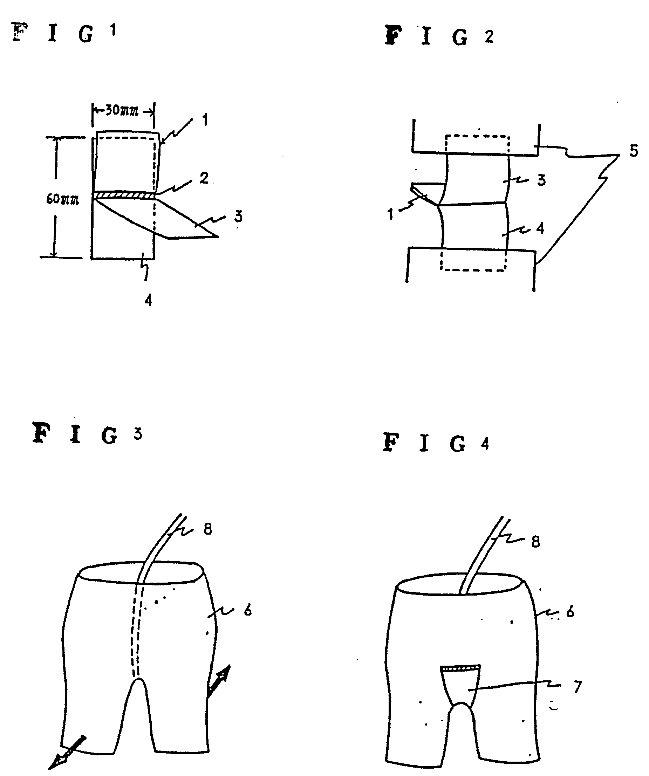

Fig. 1 is a perspective view of a sample for measurement of the bonding strength; Fig. 2 is a perspective view thereof under measurement; Fig. 3 is a perspective view of a movable female waist model for measurement of the amount dynamic adsorption; and Fig. 4 is a view showing a sample worn on the movable female waist model.

1 .. test piece, 2 .. sealed portion, 3 .. .non-woven fabric, 4 .. laminated paper, 5 .. chuck, 6 .. movable female waist model, 7 .. measurement sample, 8 .. tube.

<Testing method of fiber and non-woven fabric >

As regards fibers shown in Table 1, the melting temperatures of the first and second resin components were measured by the following method, and the proportion of the height of an endothermic peak of the second resin component relative to that of an endothermic peak of the first resin component was calculated by the following method.

Among the items of Table 2, the tensile strength was measured as to a non-woven fabric by the following method, while the bonding strength and bonding state are measured by the following method after the non-woven fabric and a leakproof sheet were subjected to the following sealing operation. The same sealing operation was made as to the absorbent article to integrate the non-woven fabric and the leakproof sheet, and the amount of dynamic absorption was measured by the following method.

(1) Melting temperature

As to the fibers as mentioned in Table 1, it is measured by the following method. The temperature of the endothermic peak observed when a sample is heated up at a rate of 10°C/min using DSC is defined as the melting temperature.

(2) Proportion of endothermic peak of second resin component relative to that of first resin component

When the height of the endothermic peak of the first resin component is $H_1$ and the height of the endothermic peak of the second resin component is $H_2$, these values are read from a DSC chart, and the above-mentioned proportion is calculated by the equation $(H_2/H_1) \times 100$ (%).

(3) Tensile strength

A non-woven fabric having a width of 50 mm and a length of 150 mm is pinched and pulled at a pulling rate of 300 mm/min. The breaking strength is defined as the value of tensile strength. The fiber orientation of the non-woven fabric is in the width direction of the non-woven fabric sample.

(4) Sealing operation

6

As to absorbent articles as shown in Table 2, a non-woven fabric and a leakproof sheet (modified polyethylene-laminated paper) are heat-sealed with a sealing width of 2 mm at a line speed of 30 m/min.

(5) Bonding strength

A test piece 1 as shown in Fig. 1 which includes a sealing portion 2 and has a width of 30 mm is cut from a sample after the sealing test. As shown in Fig. 2, the end portion of the non-woven fabric 3 and the end portion of the laminated paper 4 are respectively pinched by chucks 5 and pulled. The maximum peeling load is defined as the bonding strength.

(6) Bonding state

A sealing portion is visually observed to evaluate the bonding state. The rating criteria are as follows.
Third grade ... There is neither breakage in a heat bonded portion nor adhesion to a sealer.
Second grade ... There is a partial breakage or incomplete bonding but no adhesion to the sealer.
First grade ... There are breakage in a heat-bonded portion and adhesion to the sealer. Thus, heat bonding is impossible.

(7) Amount of dynamic absorption

A measurement sample 7 is put on a movable female waist model 6 as shown in Fig. 3 in such a way as shown in Fig. 4. After it is started, a test liquid is injected through a tube 8 into the sample at a rate of 15 g/min while continuing walking movement. The amount of the test liquid injected when leakage is confirmed is defined as the amount of dynamic absorption. The larger the amount of dynamic absorption, the less liable to occur the leakage.

(8) Cuttability

An absorbent article subjected to the sealing operation is cut with a rotary die cutter while passing it at a line speed of 30 m/min to evaluate the cuttability. The rating criteria are as follows.
Third grade ... The cut portion is completely cut.
Second grade ... Part of the cut portion remains uncut.
First grade ... The portion to be cut is hardly cut.

Table 1

| Fiber | Ref. No. | Maker | First resin component | | Second resin component | | |
|-------|----------|-------|-----------------------|---|------------------------|---|---|
| | | | Composition | Melting Temp. °C | Composition | Melting Temp. °C | Proportion of height of endothermic peak of second resin relative to that of first resin |
| PET-1 | PET-1 | Teijin | Polyethylene terephthalate | 251 | Low-crystallinity polyester | 133 | 5 |
| PET-2 | PET-2 | Teijin | Polyethylene terephthalate | 255 | Low-crystallinity polyester | 129 | 9 |
| PET-3 | PET-3 | Teijin | Polyethylene terephthalate | 256 | Low-crystallinity polyester | 132 | 18 |
| PET-4 | PET-4 | Teijin | Polyethylene terephthalate | 250 | Low-crystallinity polyester | 134 | 39 |
| PET-5 | PET-5 | Teijin | Polyethylene terephthalate | 251 | Low-crystallinity polyester | 134 | 55 |
| PET-6 | PET-6 | Teijin | Polyethylene terephthalate | 225 | Low-crystallinity polyester | 133 | 7 |
| Melty | PET-7 | Unitika | Polyethylene terephthalate | 255 | Amorphous polyester | No peak observed | 0 |
| NBF(SH) | SH | Daiwa Spinning | Polyester | 265 | Polyethylene | 133 | 212 |
| NBF(H) | NBF | Daiwa Spinning | Polypropylene | 161 | Polyethylene | 133 | 262 |
| PP | PP | Daiwa Spinning | Polypropylene | 163 | | | |
| PET | PET | Teijin | Polyethylene terephthalate | 268 | | | |

(Note) Low-crystallinity polyester and amorphous polyester are made of polyethylene terephthalate.

EP 0 270 846 B1

Table 2

| Ex. No. and Comp. Ex. No. | Web stabilization method | Areal weight g/m² | Non-woven fabric | | | | | | | | Absorbent article | | | | | |
| | | | Surface layer | | | | Reverse layer | | | | Tensile strength g/50mm | Bonding strength g/30mm | Bonding state grade | Amount of dynamic absorption g | Processing temp. °C | Cuttability grade |
| | | | Areal weight g/m² | Fiber | Fineness denier | Mixing rate % | Areal weight g/m² | Fiber | Fineness denier | Mixing rate % | | | | | | |
| Ex. 1 | heat bonding | 27 | 12 | PET-1 | 3 | 100 | 15 | PET-1 PET | 3 6 | 50 50 | 125 | 101 | 3 | 7.6 | 220 | 3 |
| Ex. 2 | heat bonding | 27 | 12 | PET-2 | 3 | 100 | 15 | PET-2 PET | 3 6 | 50 50 | 149 | 120 | 3 | 8.1 | 220 | 3 |
| Ex. 3 | heat bonding | 27 | 12 | PET-3 | 3 | 100 | 15 | PET-3 PET | 3 6 | 50 50 | 192 | 155 | 3 | 9.1 | 220 | 3 |
| Ex. 4 | heat bonding | 27 | 12 | PET-4 | 3 | 100 | 15 | PET-4 PET | 3 6 | 50 50 | 223 | 188 | 3 | 8.0 | 220 | 3 |
| Ex. 5 | heat bonding | 27 | 12 | PET-5 | 3 | 100 | 15 | PET-5 PET | 3 6· | 50 50 | 268 | 203 | 3 | 8.0 | 220 | 3 |
| Comp. Ex. 1 | heat bonding | 27 | 12 | PET-6 | 4 | 100 | 15 | PET-7 PET | 4 6 | 50 50 | 66 | 35 | 2 | 5.1 | 220 | 3 |
| Comp. Ex. 2 | heat bonding | 27 | 12 | SII | 2 | 100 | 15 | SII PET | 2 6 | 50 50 | 301 | 156 | 3 | 7.8 | 220 | 1 |
| Comp. Ex. 3 | heat bonding | 27 | 12 | NBF | 2 | 100 | 15 | NBF PET | 2 6 | 50 50 | 330 | 99 | 2 | 6.4 | 160 | 1 |
| Comp. Ex. 4 | fluid inter-locking | 40 | | PET-5 PET | 3 2 | 0 100 | | | | | 220 | 0 | not bonding | 4.3 | 220 | 3 |
| Comp. Ex. 5 | fluid inter-locking | 40 | | PET-5 PET | 3 2 | 25 72 | | | | | 206 | 39 | 3 | 4.6 | 220 | 3 |

Table 2 (continued)

| Ex. No. and Comp. Ex. No. | Non-woven fabric | | | | | | | | | | | | Absorbent article | | | |
|---|---|---|---|---|---|---|---|---|---|---|---|---|---|---|---|---|
| | Web stabilization | Areal weight g/m² | Surface layer | | | | Reverse layer | | | | Tensile strength g/50mm | Bonding strength g/30mm | Bonding state grade | Amount of dynamic absorption g | Processing temp. °C | Cuttability grade |
| | | | Areal weight g/m² | Fiber | Fineness denier | Mixing rate % | Areal weight g/m² | Fiber | Fineness denier | Mixing rate % | | | | | | |
| Ex. 6 | fluid interlocking | 40 | | PET-5 PET | 3 2 | 40 60 | | | | | 232 | 75 | 3 | 7.0 | 220 | 3 |
| Ex. 7 | fluid interlocking | 40 | | PET-5 PET | 3 2 | 80 20 | | | | | 222 | 201 | 3 | 9.5 | 220 | 3 |
| Ex. 8 | fluid interlocking | 40 | | PET-5 PET | 3 2 | 100 0 | | | | | 229 | 255 | 3 | 8.3 | 220 | 3 |
| Comp. Ex. 6 | heat bonding | 27 | | PET-5 SH | 3 2 | 0 100 | | | | | 201 | 231 | 3 | 8.8 | 220 | 1 |
| Comp. Ex. 7 | heat bonding | 27 | | PET-5 SH | 3 2 | 30 70 | | | | | 215 | 222 | 3 | 7.6 | 220 | 2 |
| Ex. 9 | heat bonding | 27 | | PET-5 SH | 3 2 | 40 60 | | | | | 213 | 212 | 3 | 8.1 | 220 | 3 |
| Ex. 10 | heat bonding | 27 | 12 | PET-5 SH | 3 2 | 70 30 | | | | | 241 | 219 | 3 | 8.2 | 220 | 3 |
| Comp. Ex. 8 | heat bonding | 27 | 12 | PET-5 | 3 | 100 | 15 | PP | 2 | 100 | 235 | 226 | 3 | 8.2 | 220 | 1 |
| Ex. 11 | heat bonding | 27 | 12 | PET-5 | 3 | 100 | 15 | PP PET-5 | 2 3 | 70 30 | 241 | 234 | 3 | 7.9 | 220 | 3 |
| Ex. 12 | heat bonding | 27 | 12 | PET-7 | 3 | 100 | 15 | PET-6 PET | 3 6 | 50 50 | 225 | 203 | 3 | 7.6 | 200 | 3 |

[Effects of the Invention]

As is apparent from Examples 1 to 12, the non-woven fabric according to the present invention provides a high bonding strength and a good bonding state when thermally processed, and has a very good cuttability. The absorbent article obtained by integrating the non-woven fabric and the leakproof layer by heat bonding shows a large amount of dynamic absorption.

In Comparative Example 1, since the proportion of an endothermic peak of a second resin component, PET-7, relative to that of a first resin component is 0%, the bonding strength by thermal processing is poor, the amount of dynamic absorption of the absorbent article is small, and the strength of the non-woven fabric is poor.

Comparative Examples 2 and 3 are good in thermal processing, but the cuttability is so poor that high-speed production may be impossible, since the second resin component is a polyolefin.

Comparative Examples 4 and 5 have a good cuttability but their bonding strength is unpractically low because the blending ratio of PET to ImpPET according to the present invention is low.

Comparative Examples 6 and 7 have a good thermal processability but a poor cuttability in contrast with Examples 4 and 5 because the blending rate of PET to ImpPET according to the present invention is small and a polyolefin fiber is mainly used.

Comparative Example 8 has a poor cuttability because the content of polyolefin, PP, is 100% in the reverse surfave layer.

## Claims

1.  An absorbent article comprising, as the surface material, a non-woven fabric containing 40 wt.% or more of a conjugate fiber made of a first polyester and a second polyester having a melting temperature of 50° C or more below that of said first polyester and a height of an endothermic peak of 5 % or more of that of the first polyester.

## Revendications

1.  Article absorbant comprenant, comme matière de surface, une étoffe non tissée contenant 40 % en poids ou plus d'une fibre conjuguée constituée d'un premier polyester et d'un second polyester ayant une température de fusion inférieure de 50° C ou plus à celle du premier polyester et une hauteur de pic endothermique valant 5 % ou plus de celle du premier polyester.

## Patentansprüche

1.  Absorptionsartikel, dadurch gekennzeichnet, daß als Oberflächenmaterial ein Vliesstoff vorgesehen ist mit einem Gehalt von 40 Gew.-% oder mehr einer Zweikomponentenfaser, hergestellt aus einem ersten Polyester und einem zweiten Polyester mit einer Schmelztemperatur von 50° C oder mehr, unterhalb derjenigen des ersten Polyesters und einer Höhe eines endothermen Spitzenwertes von 5 % oder mehr desjenigen des ersten Polyesters.

FIG 1

FIG 2

FIG 3

FIG 4